# EUROPEAN PATENT APPLICATION

(11) **EP 1 050 582 A2**
(43) Date of publication of application: **08.11.2000**
(21) Application number: 99112205.2
(22) Date of filing: 25.06.1999
(51) Int. Cl.: C12N 15/29, C07K 14/415, C12N 15/54, C12N 9/10, C12N 15/62, C12N 5/10, C12N 1/21, C08F 36/08

(54) **Recombinant microorganisms expressing small rubber particle-associated protein**

(30) Priority: 03.05.1999 KR 9915862
(71) Applicant: Korea Kumho Petrochemical Co. Ltd., Jongro-gu, Seoul 110-110 (KR); Malaysian Rubber Board, 50716 Kuala Lumpur (MY)
(72) Inventor: Han, Kyung-Hwan, Kwangsan-gu, Kwangju 506-302 (KR); Kang, Hunseung, Kwangju 500-170 (KR); Oh, Soo Kyung, Sungnam, Kyounggi-do 463-070 (KR); Chow, Keng See, Biotech. and Strat. Research Unit, Kuala Lumpur (MY); Faridah, Yusof, Biotech. and Strat. Research Unit, Kuala Lumpur (MY); Yeang, Hoong Yeet, Biot. and Strat. Research Unit, Kuala Lumpur (MY)
(74) Representative: Godemeyer, Thomas, Dr.

(57) **Abstract**

The present invention relates to cDNA encoding small rubber particle-associated protein(SRPP) which binds to small rubber particle derived from the latex of *H*. *brasiliensis,* recombinant SRPP deduced from the cDNA, recombinant expression vectors comprising the cDNA, recombinant microorganisms transformed with the recombinant expression vectors and method for synthesis of rubber using the recombinant SRPP. The recombinant SRPP expressed in the recombinant microorganisms can be applied for the rubber synthesis in the presence of rubber particle.

## Description

### Field of the Invention

The present invention relates to recombinant microorganisms expressing small rubber particle-associated protein("SRPP"), more specifically, cDNA encoding the SRPP derived from the latex of *Hevea brasiliensis,* recombinant SRPP deduced from the cDNA, recombinant expression vectors comprising the cDNA, recombinant microorganisms transformed with the recombinant expression vectors and method for synthesis of rubber using the recombinant SRPP.

### Background of the Invention

Rubber(*cis*-1,4-polyisoprene), an isoprenoid polymer with no known physiological function to the plant, is produced in about 2,000 plant species with varying degrees of quality and quantity (see: Backhaus, R.A., Israel Journal of Botany, 34:283-293(1985)). The rubber is a raw material of choice for heavy-duty tires and other industrial uses requiring elasticity, flexibility and resilience. *Hevea brasiliensis* ("*H. brasiliensis")* has been regarded as the only commercial source of natural rubber mainly due to its abundance in the tree, its quality and the ease with harvesting. Diminishing acreage of rubber plantation and life-threatening latex allergy to *Hevea* rubber, coupled with increasing demand, have concentrated research interests in the area of rubber synthesis and development of an alternative rubber source.

In *H. brasiliensis,* rubber synthesis takes place on the surface of rubber particles suspended in the latex(the cytoplasm of laticifers). The laticifers are specialized vessels that are located adjacent to the phloem of the rubber tree. When severed during tapping, the high tugor pressure inside the laticifers expels latex containing 30-50%(wt/wt) of *cis*-1,4-polyisoprene. The latex may be fractionated by centrifugation into three phases: the top fraction containing mostly rubber particles; the middle fraction (called C-serum) which is metabolically active fraction; and, the bottom fraction with mainly vacuoles called lutoids.

More than 200 expressed sequence tags have been identified from the latex of *H. brasiliensis.* Kush et al. have shown differential expression of several rubber biosynthesis-related genes in latex (see: Kush, A. et al., Proc. Natl. Acad. Sci., U.S.A., 87:1787-1790(1990)). The rubber elongation factor("REF"), an enzyme involved rubber biosynthesis, is highly expressed in laticifers (see: Dennis, M.S. and Light, D.R., J. Biol. Chem., 264:18608-17(1989); Goyvaerts, E. et al., Plant Physiol., 97:317-321(1991)). Laticiferous cells actively translate the transcribed genes into proteins. Tupy et al. reported the presence of ribosomes and polysomes in these specialised cells (see: Tupy et al., Plant Science, 55:137-144(1988)). Arokiaraj et al. observed that β-glucuronidase reporter gene introduced by *Agrobacterium*-mediated transformation was expressed in the latex of transgenic *Hevea* plant (see: Arokiaraj et al., Plant Cell Reports, 17:621-625(1998)).

Genes expressed in the latex of *Hevea* can be divided into three groups based on their encoding proteins: 1) Defense-related proteins such as hevein, chitinase, β-1,3-glucanase, and *HEVER* (see: Broeckaert et al., Proc. Natl. Acad. Sci., U.S.A., 87:7633-7637(1990); Martin, M.N., Plant Physiology, 95:469-476(1991); Chye, M.L. and Cheung, K.Y., Plan Mol. Biol., 29:397-402(1995); Sivasubramanism, S. et al., Plant Mol. Biol., 29:173-178(1995)); 2) Rubber biosynthesis-related proteins such as REF, HMGCoA reductase, and farnesyl diphosphate synthase (see: Goyvaerts, E. et al., Plant Physiol., 97:317-321(1991); Chye, M.L. et al., Plant Mol. Biol., 19:473-484(1992); Adiwilaga, K. and Kush, A., Plant Mol. Biol., 30:935-946(1996)); and, 3) Latex allergens (proteins) such as Hev b 3, Hev b 4, Hev b 5 and Hev b 7(see: Yeang, H.Y. et al., Journal of Allergy and clinical Immunology, 98:628-639(1996); Akasawa, A. et al., J. Biol. Chem., 271:25389-93(1996); Slater, J.E. et al., J. Biol. Chem., 271:25394-9(1996); Sowka, S. et al., Eur. J. Biochem., 255:213-9(1998)).

Rubber particles are essential components in rubber synthesis *in vitro* (see: Lynen, F., J. Rub. Res. Inst. Malaya, 21:389-406(1969)). So far, most of the experiments showing *in vitro* rubber synthesis with labeled isopentenyl pyrophosphate ("IPP") have always required the presence of rubber particles. Dennis and Light had shown that REF, a kind of rubber particle-associated protein("RPP"), was necessary for rubber elongation *in vitro*(see: Dennis, M.S. and Light, D.R., J. Biol. Chem., 264:18608-17(1989)). Yeang et al. identified a 24kDa RPP(Hev b 3) tightly bound on small rubber particles smaller than 10µm in diameter as another latex allergen(see: Yeang, H.Y. et al., Journal of allergy and clinical immunology, 98:628-639(1996); Yeang, H.Y. et al., 53:513-519(1998)). In addition, Pan et al. identified the most abundant RPP(53kDa) in guayule, another rubber-producing plant, as a cytochrome P450 known as allene oxide synthase (see: Pan, Z. et al., J. Biol. Chem., 279:8487-8494(1995)), which is also reported as an essential protein for rubber biosynthesis(see: USP 5,633,433).

In light of these observations, it is plausible that the surface of rubber particle is the appropriate location for a rubber polymerase, which plays a role in polymerizing a hydrophobic polymer into the particle interior while obtaining hydrophilic substrates from the cytosol. As major RPPs, both REF and SRPP could be the integral components of the rubber biosynthesis machinery.

SRPP causes allergenic response in spina bifida patients and only its partial amino acid sequence has been determined(see: Yeang, H.Y. et al., Journal of allergy and Clinical Immunology, 98:628-639(1996); Yeang, H.Y. et al., Allergy, 53:513-519(1998)), while the gene encoding this protein has not been cloned yet. Isolation and characterization of the SRPP gene have accelerated further study on the functions and industrial uses of SRPP.

### Summary of the Invention

In accordance with the present invention, the inventors have made an effort to elucidate the functions of SRPP related to rubber synthesis, and cloned full length cDNA coding for SRPP from latex cDNA library of *H. brasiliensis* and determined the amino acid sequence deduced from the genes. Further, the inventors constructed expression vectors comprising the SRPP cDNA and recombinant microorganisms transformed therewith, finally to confirm a positive role of SRPP in rubber synthesis.

The first object of the present invention, therefore, is to provide cDNA coding for SRPP.

The second object of the invention is to provide amino acid sequence deduced from the cDNA.

The third object of the invention is to provide recombinant expression vectors comprising the cDNA.

The fourth object of the invention is to provide recombinant microorganisms transformed with the said expression vectors.

The fifth object of the invention is to provide a method for synthesis of rubber using the recombinant SRPP.

### Brief Description of Drawings

The above and the other objects and features of the present invention will become apparent from the following description given in conjunction with the accompanying drawings, in which:
- Figure 1: is the nucleotide sequence of SRPP cDNA(SEQ ID NO:2) and amino acid sequence deduced therefrom(SEQ ID NO:3).
- Figure 2: is a hydropathy plot calculated from the deduced amino acid sequence of SRPP.
- Figure 3: is a comparison result of the deduced amino acid sequence of SRPP with other rubber synthesis-related proteins.
- Figure 4a: is a genetic map of a recombinant expression vector pGEX-SRPP.
- Figure 4b: is a genetic map of a recombinant expression vector pRSET-SRPP.
- Figure 5a: is a graph showing the increase of [¹⁴C]-IPP incorporation depending on the concentration of SRPP.
- Figure 5b: is a graph showing the decrease of [¹⁴C]-IPP incorporation depending on the concentration of SRPP-specific antibody.

### Detailed Description of the Invention

The present inventors isolated a cDNA clone encoding 24kDa of small rubber particle-associated protein("SRPP"), known as Hev b3 protein, from latex cDNA library of *H. brasiliensis,* where the SRPP tightly binds on small rubber particle whose size is less than 10µm in diameter. Then, the amino acid sequence deduced from the cDNA was determined, which revealed that: SRPP cDNA is 910bp long, and contains 83bp 5'-UTR(untranslated region), 615bp ORF(open reading frame) and 212bp 3'-UTR including 18bp poly A tail. A putative polyadenylation signal(AATAA) beginning at 794^{th} position was also identified. The ORF encodes 204 amino acids of protein with a predicted molecular mass of approximately 22.4kDa. Analysis of the SRPP revealed that it is a hydrophobic and acidic protein with an isoelectric point of 4.8 and located in cytoplasm. The SRPP exhibits 72% sequence similarity to REF(GenBank Accession No. P15252), 68% sequence similarity to stress-related protein of *P*. *vulgaris*(PvSRP, GenBank Accession No. 1326163) and 48% sequence similarity to *Arabidopsis thaliana* FIN21.4(GenBank Accession No. 2670320), Sequence alignment indicates that there is highly conserved regions from amino acid number 38 to 65 with no match to any known functional domains.

Recombinant expression vectors pGEX-SRPP and pRSET-SRPP provided by this invention were constructed by cloning of the 910bp SRPP cDNA either in the *EcoR*I-*Xho*I site of pGEX or in the *BamH*I-*Pvu*II site of pRSET, In the pGEX-SRPP, glutathione S-transferase(GST)-SRPP fusion protein is expressed under the control of isopropyl-β-thiogalacto pyranoside(IPTG)-inducible *tac* promoter; and, in the pRSET-SRPP, SRPP is independently expressed under the control of T7 promoter. Each of transformants thus prepared was designated as *'Escherichia coli* XL1 Blue/pGEX-SRPP' and *'Escherichia coli* XL1 Blue/pRSET-SRPP', and deposited with the Korean Collection for Type Cultures(KCTC, #52, Oun-dong, Yusong-gu, Taejon 305-333, Republic of Korea), an international depository authority as accession No. KCTC 0595BP and KCTC 0596BP on March 30, 1999.

In accordance with the conventional method, the recombinant *E*. *coli* XL1 Blue/pGEX-SRPP or XL1 Blue/pRSET-SRPP was grown in Luria Broth("LB") media containing ampicillin, homogenated after IPTG induction, centrifuged and the supernatant containing recombinant SRPP was collected.

Rubber can be easily produced *in vitro* by the addition of the recombinant SRPP thus obtained to a rubber synthesis reaction mixture containing rubber particles. Preferably, rubber can be manufactured by adding *Hevea brasiliensis* latex or washed rubber particle(WRP) and the recombinant SRPP to a reaction buffer solution where protein degradation is inhibited, and incubating at 20 to 37°C for 2 to 12 hours. Under the consideration of manufacturing efficiency, the addition of concentrated recombinant SRPP is more preferable since productivity is highly dependent on the concentration of recombinant SRPP. The recombinant SRPP can be expressed in a host cell of a plant, a bacteria, a yeast or a fungus which is transformed with the expression vectors comprising a cDNA encoding SRPP(SEQ ID NO:2) in a form of fusion protein with another protein or non-fusion protein. Both SRPP fusion protein and non-fusion protein may be used for rubber synthesis reaction. The supernatant obtained by centrifugation of the host cell homogenate may directly be used for the rubber synthesis without further purification.

The present invention is further illustrated by the following examples, which should not be taken to limit the scope of the invention.

### Example 1: Construction of cDNA library of H. brasiliensis latex

### Example 1-1: Construction of cDNA library

Latex and leaves were obtained from mature rubber plants(*H*. *brasiliensis* RRIM600) growing at the Rubber Research Institute of Malaysia, Selangor, Malaysia. Latex exuding from the tapped trees was collected while continuously mixing it with an equal volume of 2X RNA extraction buffer(0.1M Tris HCl, pH 9.5, containing 0.3M LiCl, 0.01M EDTA, 10% SDS) either at ambient temperature or on ice. The collected samples were frozen in liquid nitrogen.

RNA extraction from latex was performed analogously as in Kush et al's method (see: Kush, A. et al., Proc. Natl. Acad. Sci., U.S.A., 87:1787-1790(1990)) using the Qiagen RNeasy Plant Minikit(Qiagen Inc., U.S.A.). Poly (A)⁺ RNA was isolated using Oligotex-dT™ mRNA Kit(Qiagen Inc., U.S.A.). The cDNA library was constructed in an Uni-ZAP II phage vector according to the supplier's instructions (Stratagene, U.S.A.) using Poly(A)⁺ RNA prepared from the latex. As a result, the cDNA library containing about 5 × 10⁷ recombinant phages was constructed.

### Example 1-2: Screening of cDNA library

In a subtracted cDNA library(latex leaf), the inventors identified an expressed sequence tag(EST) whose deduced amino acid matches with the known partial sequence of Hev b 3. From the sequence of the EST, a PCR primer(see: SEQ ID NO:1) corresponding to the sequences from +185 to +211 bp in SRPP was designed. This primer(upstream) and T7 primer(downstream) were used to amplify SRPP genes from the cDNA library obtained from Example 1-1. PCR was performed for 30 cycles of 30sec at 94°C, 30sec at 50°C, and 2min at 72°C, with a 5min pre-heat and 10min final extension at 72°C. The 500bp-SRPP DNA fragment obtained from the PCR was employed as a probe for the screening of cDNA library which is diluted by a factor of 10². From the screening, 10 positive clones containing about 0.8 to 1.0kb insert DNA were detected from agarose gel electrophoresis after digestion with *EcoR*I. One clone carrying approximately 1.0Kb DNA insert was selected for subcloning. According to the supplier's instruction of cDNA library kit(Stratagene, U.S.A.) used in Example 1-1, phagemid containing 1.0kb DNA was excised from the Uni-ZAP II phage vector. The phagemid was designated as "pSRPP".

### Example 1-3: Sequence analysis of cDNA clone

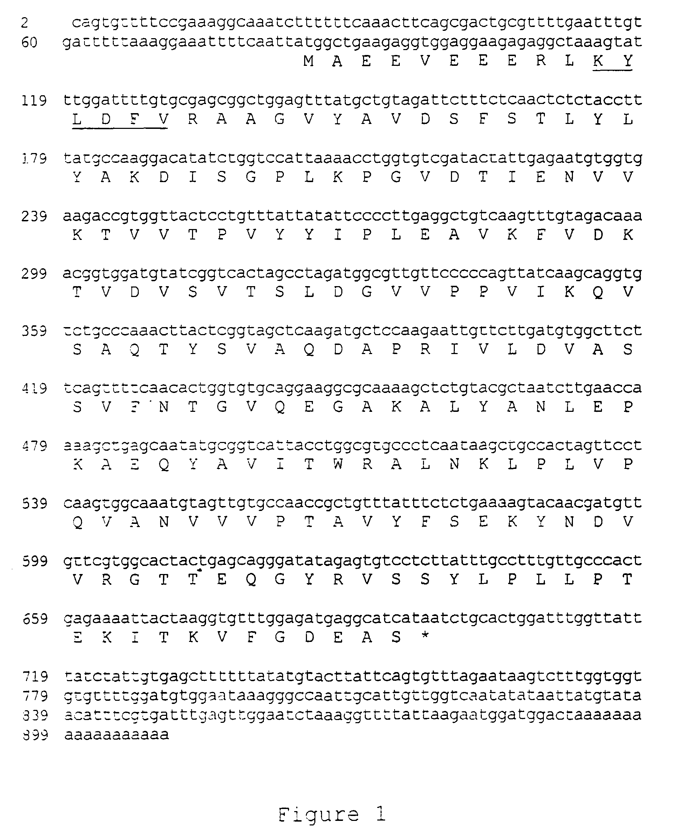

For the cDNA sequence analysis of the pSRPP, plasmid DNA of *E*. *coli* XL-1 Blue/pSRPP was prepared by the alkaline lysis method (see: Sambrook J. et al., Molecular cloning, A laboratory manual, 2^{nd} edition: Cold Spring Harbor Laboratory Press(1989)) using Wizard® Plus SV Minipreps DNA Purification System kit(Promega, U.S.A.). The sequencing reaction was performed with the ALFexpress AutoRead Sequencing kit(Pharmacia Biotech, Sweden) using the fluorescent dye-labeled M13 universal or reverse primer(provided by the kit). The nucleotide sequences were obtained by electrophoresis on an ALF automatic sequencer(Perkin-Elmer, U.S.A.). The sequence analysis showed that DNA fragment inserted to pSRPP is 910bp long(SEQ ID NO:2) and contains 83bp 5'-UTR, 615bp ORF and 212bp 3'-UTR including poly A tail of 18bp. A putative polyadenylation signal (AATAA) beginning at bp 794^{th} position was also identified (see: Figure 1) .

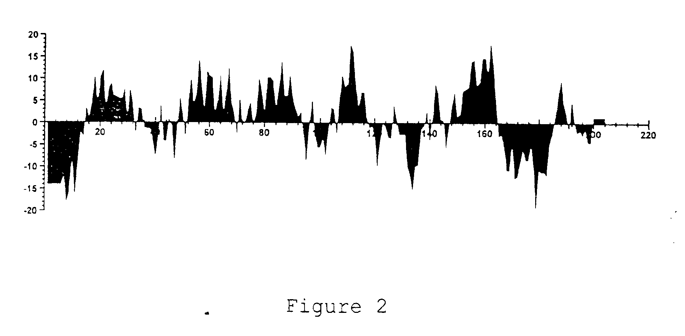

The nucleotide sequence(SEQ ID NO:2) encodes 204-amino acids(see: SEQ ID NO:3) with a predicted molecular mass of 22.4kDa (see: Figure 1). The deduced protein is acidic with an isoelectric point of 4.8 which is similar to that of REF(pI=5.04). Hydropathy analysis of the deduced amino acid sequence revealed that SRPP has a hydrophobic nature(see: Figure 2). Transmembrane region analysis using TMpred algorithm indicated that the SRPP does not have transmembrane helices large enough to span the lipid bilayers. A computer analysis by the PSORT program(K. Nakai, Osaka University, Japan) for protein localization sites suggests that the SRPP is localized in cytoplasm (certainty=0.65).

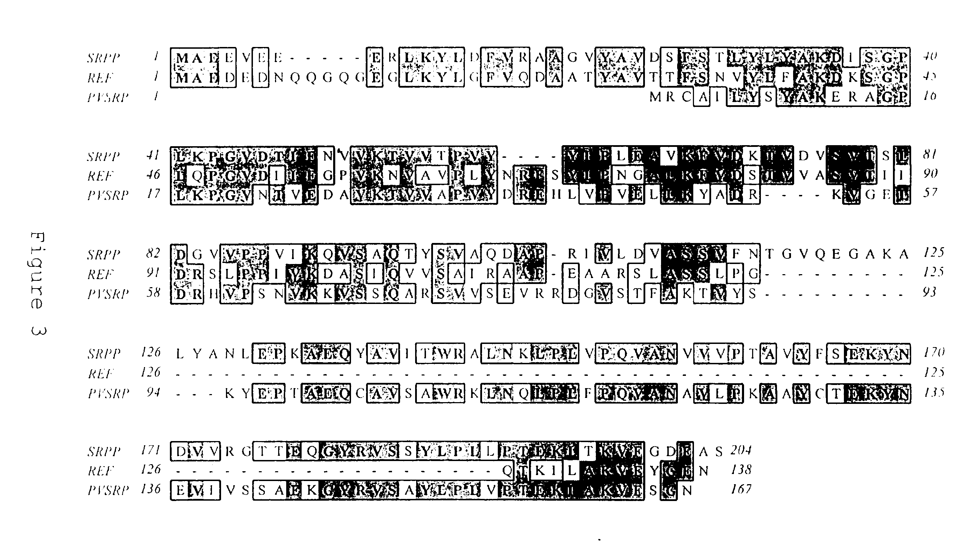

The computer analysis using Clustal W program shows that the predicted amino acid sequence of the SRPP has a high similarity to those of REF(72% in the paired sequence region, accession No. P15252), *P*. *vulgaris* stress-related protein(PvSRP, 68% in the paired sequence region, accession No. 1326163), and *A. thaliana* FIN21.4(48% in the paired sequence region, accession No. 2670320). PvSRP is believed to be involved in defense mechanism and induced by stress or wounding. The function of the *Arabidopsis* gene FIN21 is not known. Sequence alignment indicates that there is a highly conserved region from the amino acid number 38 to 65 with no match to any known functional domains(see: Figure 3).

### Example 2: Expression of recombinant SRPP

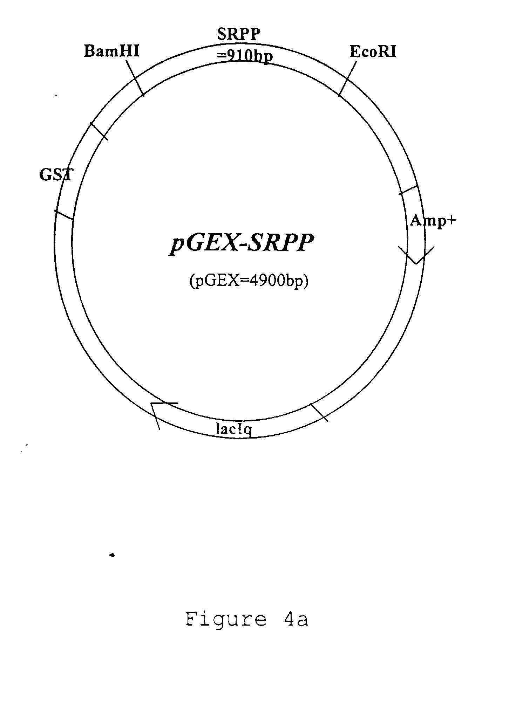

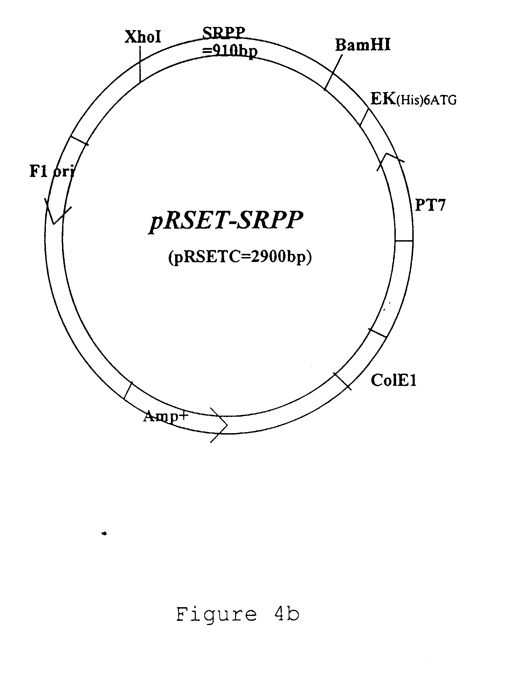

The cDNA of SRPP was excised from the pSRPP of Example 1-3 with *EcoR*I and *Xho*I for construction of SRPP expression vector. Recombinant expression vector pGEX-SRPP or pRSET-SRPP was constructed by cloning of the SRPP cDNA either in the *EcoR*I-*Xho*I site of pGEX(Pharmacia, Sweden) or in the *BamH*I-*Pvu*II site of pRSET(Invitrogen, U.S.A.)(see: Figures 4a and 4b). In the pGEX-SRPP, glutathione S-transferase(GST)-SRPP fusion protein is expressed under the control of isopropyl-β-thiogalactopyranoside(IPTG)-inducible *tac* promoter and in the pRSET-SRPP, SRPP is independently expressed under the control of T7 promoter. Each of transformants thus prepared was designated as *'Escherichia coli* XL1 Blue/pGEX-SRPP' and *'Escherichia coli* XL1 Blue/pRSET-SRRPP', and deposited with the Korean Collection for Type Cultures(KCTC, #52, Oun-dong, Yusong-gu, Taejon 305-333, Republic of Korea), an International depository authority as accession No. KCTC 0595BP and KCTC 0596BP on March 30, 1999.

*E. coli* XL1-Blue transformed with either pGEX-SRPP or pRSET-SRPP was grown to mid-stationary phase in LB containing 50mg/L ampicillin at 30°C with vigorous aeration; induced by adding IPTG to reach a concentration of 0.1mM; and, incubated for another 4hrs. All subsequent steps were carried out at 4°C. The cells were harvested, washed with 0.1M potassium phosphate(pH 7.4) by centrifugation (5000×g, 10min), and then disrupted by sonication. The cell homogenate was centrifuged at 10,000×g for 10min and the supernatant was subjected to SDS-PAGE according to Laemmli's method(see: Laemmii, U.K., Nature, 277:680-685(1970)), and the proteins were stained by Coomassie Brilliant Blue R-250. Hereinafter, the supernatant separated from the cell homogenates is referred to as "cell extracts".

As a result, approximately 48kDa GST-SRPP fusion protein from *E.coli* XL-1 Blue/pGEX-SRPP and approximately 24kDa recombinant SRPP from *E.coli* XL-1 Blue/pRSET-SRPP were expressed, respectively.

### Example 3: Synthesis of rubber using recombinant SRPP

To investigate whether SRPP is related to rubber synthesis, *in vitro* rubber synthesis was performed using *Hevea* C-serum and washed rubber particle(WRP): WRPs were first prepared by repeating centrifugation/floatation procedure(see: Cornish, K. and Backhaus, R.A., Phytochemistry, 29:3809-3813(1990); Siler, D.J. and Cornish, K., Phytochemistry, 32:1097-1102(1993)). The whole latex from *H. brasiliensis* was mixed with equal volume of a washing buffer solution(100mM Tris-HCl, pH 7.5, containing 5mM MgSO₄, 10mM DTT and 0.1mM PMSF) and centrifuged at 40,000×g for 2hrs. The clear fluid was collected as a C-serum, and the rubber particles at top layer were recovered and washes several times with the same buffer to obtain WRP. Then, rubber was synthesized *in vitro* by the conventional method in the art(see: Cornish, K. and Backhaus, R.A., Phytochemistry, 29:3809-3813(1990); Siler, D.J. and Cornish, K., Phytochemistry, 32:1097-1102(1993)). 10mg of WRP and cell extracts prepared from recombinant *E*. *coli* XL-1 Blue/pGEX-SRPP expressing GST-SRPP fusion protein in Example 2 were added in a reaction buffer(100mM Tris-HCl, pH 7.5, containing 80µM [¹⁴C]-isopentenyl pyrophosphate(IPP, 55mCi mmol⁻¹), 20µM farnesyl pyrophosphate(FPP), 1mM MgSO₄, and 1mM dithiothreitol(DTT)) to reach a reaction volume of 50 µl, and incubated at 25 °C for 6hrs. As a control, either the same cell extracts without IPTG induction or the culture of *E*. *coli* harboring pGEX expressing the GST protein was used. The reaction was stopped by adding 25mM of EDTA.

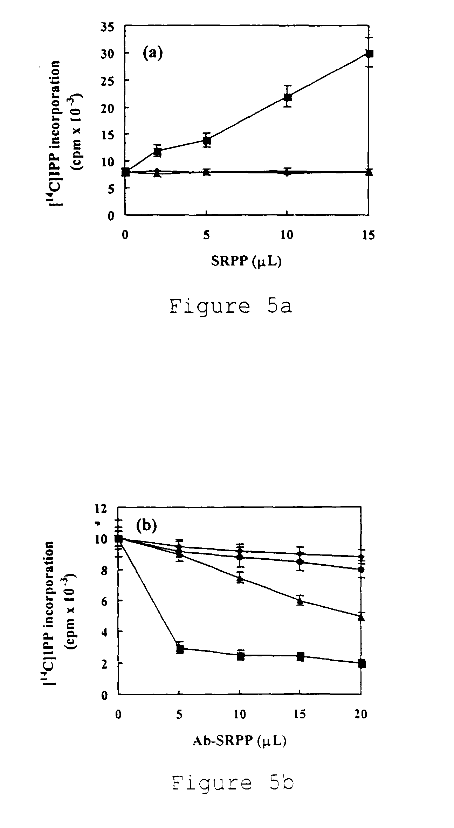

The resulting [¹⁴C]IPP-incorporated rubber was quantified by the aid of filtration or benzene extraction method: For filtration method, the reaction mixture was filtered through anodisc membrane having a pore size of 0.02 to 0.1µm(Whatman, U.S.A.), the filter was subjected to repeated washing with 1M HCl and 95% ethanol(see: Cornish, K. and Backhaus, R.A., Phytochemistry, 29:3809-3813(1990)), and the remaining-radioactivity on the washed filters was quantified by a liquid scintillation counter(Beckman, U.S.A.). For benzene extraction method, the reaction mixture was extracted three times with two volumes of benzene, the benzene extract was mixed with a Ready Solv HP scintillation cocktail (Beckman, U.S.A.) and the radioactivity was determined by a liquid scintillation counter. The [¹⁴C] IPP-incorporated rubber was increased depending on the concentration of SRPP(see: Figure 5a). In Figure 5a, (■) represents cell extracts of recombinant *E*. *coli* expressing GST-SRPP; (◆) represents cell extracts of recombinant *E*. *coli* without IPTG induction; and, (▲) represents cell extracts of recombinant *E*. *coli* expressing only the GST protein.

### Example 4: Inhibition of rubber synthesis by anti-SRPP antibody

To further confirm that SRPP plays a critical role in rubber synthesis, polyclonal antibody from SRPP-challenged rabbit serum and monoclonal antibody from mouse were prepared, respectively, and their ability to inhibit rubber synthesis in C-serum was tested, while employing a control of the untreated blood serum. The rubber synthesis was carried out in an analogous manner as in Example 3 with the exception of adding 5µl C-serum instead of *E*. *coli* cell extracts and supplementing with antibody or serum indicated in Figure 5b. Figure 5b shows that the polyclonal monoclonal antibodies generated against SRPP protein inhibited rubber synthesis to a much greater extent than the control serum. More severe reduction in rubber synthesis was observed with polyclonal antibody than monoclonal antibody. In Figure 5b, each value is the mean of experiments for control rabbit(◆) and mouse(●) serum without antibody formation, monoclonal antibody (▲) and polyclonal antibody (■). This differential inhibitory effect may be due to the immuno-cross reactivity of the polyclonal antibody with REF, based on a high sequence homology to SRPP. The results of Examples 3 and 4 suggest that SRPP be involved in rubber synthesis.

As clearly illustrated and demonstrated as aboves, the present invention provides cDNA encoding small rubber particle-associated protein(SRPP) which binds to small rubber particle derived from the latex of Hevea *brasiliensis,* recombinant SRPP deduced from the cDNA, recombinant expression vectors comprising the cDNA, recombinant microorganisms transformed with the recombinant expression vectors and method for synthesis of rubber using the recombinant SRPP. The recombinant SRPP expressed in the recombinant microorganisms can be applied for the rubber synthesis in the presence of rubber particle.

## Claims

1. A cDNA(SEQ ID NO:2) encoding small rubber particle-associated protein(SRPP) which binds to small rubber particle whose size is less than 10µm in diameter, and is derived from latex of *Hevea brasiliensis.*

2. A recombinant small rubber particle-associated protein(SRPP) which has an amino acid sequence(SEQ ID NO:3) deduced from the cDNA of claim 1.

3. A recombinant expression vector pGEX-SRPP comprising a cDNA(SEQ ID NO:2) which expresses glutathione-S-transferase(GST)-SRPP fusion protein.

4. A recombinant expression vector pRSET-SRPP comprising a cDNA(SEQ ID NO:2) which expresses small rubber particle-associated protein(SRPP).

5. *E*. *coli* XL-1 Blue/pGEX-SRPP(KCTC 0595BP) transformed with the recombinant expression vector pGEX-SRPP of claim 3.

6. *E*. *coli* XL-1 Blue/pRSET-SRPP(KCTC 0596BP) transformed with the recombinant expression vector pRSET-SRPP of claim 4.

7. A method for synthesis of rubber using the recombinant SRPP of claim 2, which comprises the steps of: adding latex of *Hevea brasiliensis* or washed rubber particle and the recombinant SRPP to a reaction mixture for rubber synthesis; incubating at 20 to 37°C. for 2 to 12 hours; and, collecting synthesized rubber from the reaction mixture.

8. The method for synthesis of rubber of claim 7, wherein the recombinant SRPP is expressed in a host cell transformed with an expression vector comprising the cDNA of claim 1.

9. The method for synthesis of rubber of claim 8, wherein the host is a plant, a bacterium, a yeast or a fungus.

10. The method for synthesis of rubber of claim 8, wherein the recombinant SRPP is expressed in a fusion protein or a non-fusion protein.

11. The method for synthesis of claim 7, wherein the rubber particle is less than 10 µm in diameter and derived from *Hevea brasiliensis.*

12. The method for synthesis of claim 7, wherein the reaction mixture is 100mM Tris-HCl(pH 7.5) buffer solution containing 80µM isopentenyl pyrophosphate (IPP), 20µM farnesyl pyrophosphate (FPP), 1mM MgSO₄, and 1mM dithiothreitol (DTT).


SEQUENCE LISTING

<110> Korea Kumho Petrochemical Co., Ltd.
      Malaysian Rubber Board

<120> Recombinant Microorganisms Expressing Small Rubber
      Particle-Associated Protein

<130> 990610ep

<140>
<141>

<150> KR 99-15862
<151> 1999-05-03

<160> 3

<170> PatentIn Ver. 2.1

<210> 1
<211> 27
<212> DNA
<213> Artificial Sequence

<220>
<223> Description of Artificial Sequence: synthetic
      oligonucleotide  (binds to the sequence 185 to 211
      of SEQ. ID. NO: 2)

<400> 1
caaggacata tctggtccat taaaacc                                     27

<210> 2
<211> 910
<212> DNA
<213> Hevea brasiliensis

<400> 2
cagtgttttc cgaaaggcaa atcttttttc aaacttcagc gactgcgttt tgaatttgtg 60
atttttaaag gaaattttca attatggctg aagaggtgga ggaagagagg ctaaagtatt 120
tggattttgt gcgagcggct ggagtttatg ctgtagattc tttctcaact ctctaccttt 180
atgccaagga catatctggt ccattaaaac ctggtgtcga tactattgag aatgtggtga 240
agaccgtggt tactcctgtt tattatattc cccttgaggc tgtcaagttt gtagacaaaa 300
cggtggatgt atcggtcact agcctagatg gcgttgttcc cccagttatc aagcaggtgt 360
ctgcccaaac ttactcggta gctcaagatg ctccaagaat tgttcttgat gtggcttctt 420
cagttttcaa cactggtgtg caggaaggcg caaaagctct gtacgctaat cttgaaccaa 480
aagctgagca atatgcggtc attacctggc gtgccctcaa taagctgcca ctagttcctc 540

aagtggcaaa tgtagttgtg ccaaccgctg tttatttctc tgaaaagtac aacgatgttg 600
ttcgtggcac tactgagcag ggatatagag tgtcctctta tttgcctttg ttgcccactg 660
agaaaattac taaggtgttt ggagatgagg catcataatc tgcactggat ttggttattt 720
atctattgtg agctttttta tatgtactta ttcagtgttt agaataagtc tttggtggtg 780
tgttttggat gtggaataaa gggccaattg cattgttggt caatatataa ttatgtataa 840
catttcgtga tttgagttgg aatctaaagg ttttattaag aatggatgga ctaaaaaaaa 900
aaaaaaaaaa 910

<210> 3
<211> 204
<212> PRT
<213> Hevea brasiliensis

<400> 3
Met Ala Glu Glu Val Glu Glu Glu Arg Leu Lys Tyr Leu Asp Phe Val
1               5                   10                  15

Arg Ala Ala Gly Val Tyr Ala Val Asp Ser Phe Ser Thr Leu Tyr Leu
            20                  25                  30

Tyr Ala Lys Asp Ile Ser Gly Pro Leu Lys Pro Gly Val Asp Thr Ile
        35                  40                  45

Glu Asn Val Val Lys Thr Val Val Thr Pro Val Tyr Tyr Ile Pro Leu
    50                  55                  60

Glu Ala Val Lys Phe Val Asp Lys Thr Val Asp Val Ser Val Thr Ser
65                  70                  75                  80

Leu Asp Gly Val Val Pro Pro Val Ile Lys Gln Val Ser Ala Gln Thr
                85                  90                  95

Tyr Ser Val Ala Gln Asp Ala Pro Arg Ile Val Leu Asp Val Ala Ser
            100                 105                 110

Ser Val Phe Asn Thr Gly Val Gln Glu Gly Ala Lys Ala Leu Tyr Ala
        115                 120                 125

Asn Leu Glu Pro Lys Ala Glu Gln Tyr Ala Val Ile Thr Trp Arg Ala
    130                 135                 140

Leu Asn Lys Leu Pro Leu Val Pro Gln Val Ala Asn Val Val Val Pro
145                 150                 155                 160

Thr Ala Val Tyr Phe Ser Glu Lys Tyr Asn Asp Val Val Arg Gly Thr
                165                 170                 175

Thr Glu Gln Gly Tyr Arg Val Ser Ser Tyr Leu Pro Leu Leu Pro Thr

            180                 185                 190
Glu Lys Ile Thr Lys Val Phe Gly Asp Glu Ala Ser
        195                 200